# EUROPEAN PATENT APPLICATION

(11) **EP 2 878 387 A2**
(43) Date of publication of application: **03.06.2015**
(21) Application number: 14386030.2
(22) Date of filing: 26.11.2014
(51) Int. Cl.: B08B 15/02

(54) **System comprising a cleaning decontamination and analysis device for computers or computer periperals.**

(30) Priority: 29.11.2013 GR 20130100665
(71) Applicant: Kakaras Sp., Konstantinos, 115 24 Ampelokipi Athens (GR)
(72) Inventor: Kakaras Sp., Konstantinos, 115 24 Ampelokipi Athens (GR)

(57) **Abstract**

The present Device includes equipment for cleaning, decontamination and analysis of computers, computing machines and their peripherals, with the ability of analysis and processing the elements - waste as well as electronic information data reception.

Its utility is vital in public health in a daily basis, but also becomes crucial when particular contaminations of the environment, such as biological or chemical contaminations, are present. Furthermore, it contributes in the reduction of energy consumption, and the proper function of computers, computing machines and their peripherals.

## Description

The present invention aims to solve the problem of cleaning and decontamination of computers - calculating machines and their peripherals with the ability of further analysis of data. This problem is mainly a result of dust particles that accumulate, including harmful allergenic and other toxic substances, smells and generally organic or non organic elements of any form that may accumulate and exist on or inside the calculating machine as non-manufactured material.

The elements - particles - substances, that are deposited or channeled and conveyed mainly through the cooling mechanisms, are damaging to the health of users, to the work space and to public health. Furthermore, their removal improves the computer's performance, extends its lifespan and the time between each failure, reduces energy consumption which is required primarily for their cooling, via their fans or other means and gives us data to process and deal with such as the quality of the air that is released to the user's space and the environment.

The capacity to collect and neutralize waste with their concurrent or in succession analysis provides the ability to deal with the conditions of daily use while dealing with cases that concern particular local or broader serious infections caused by chemicals or epidemic viruses, in which case cleaning and decontamination of space or area is required.

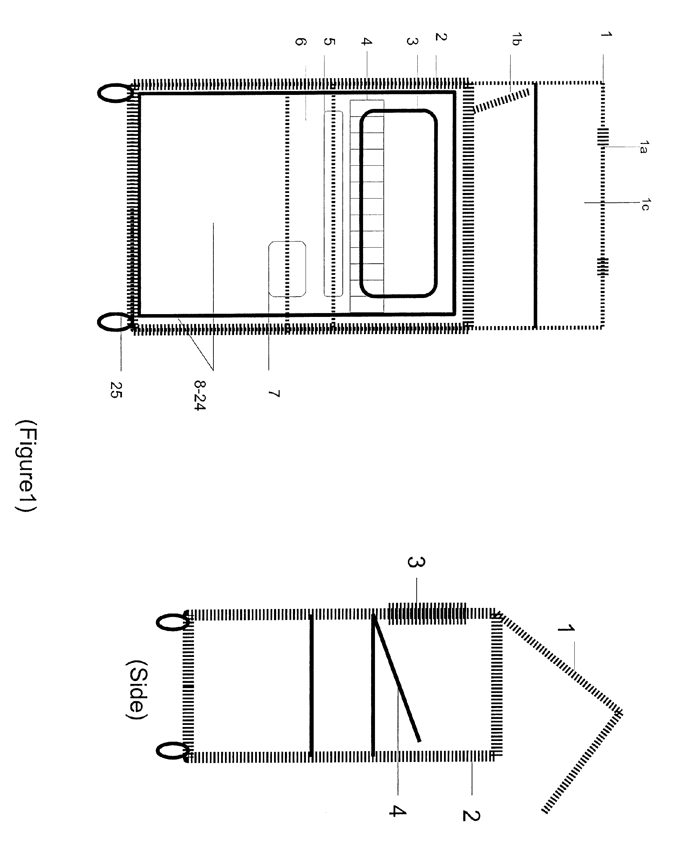

For this to be accomplished a Device (Figure1) is used (special cleaning, decontamination and analysis device) which, amongst other things, cleans by removing, absorbing, and retaining while processing the organic and non organic elements - byproducts and concurrently or in succession decontaminates the calculating machine as well as having the capacity to analyze the above and includes means of recording and processing further computer data.

The Device (Figure1) can be used - manufactured as a whole or as a part with the objective of cleaning or decontaminating or analyzing or a combination of the above.

The Device (Figure1) may be used in stable position or as portable
where needed.

The proportions and characteristics of the Device (Figure1) can be altered depending on the versions that each Device (Figure1) can have. Indicatively an example of this is cleaning, decontamination and analysis of just a portable computer, something for which a Device can be use (Figure1) according to the computer's proportions, in relation to the size which is required for a single or multiple desktop computers to be cleaned, decontaminated and analyzed in specific time.

Until today simple absorbers and air pressure devices are used for local cleaning without afterwards analysis and decontamination while in the cases of total reconstruction of devices several technologies are used such as immersing of the parts in special chemicals, and ultrasound. The latter requires specialized staff for complete deconstruction of every device that will come of it. This calls for increased cost and time.

### Short presentation of the Device

Figure1 represents the device along with its parts, which are described below from 1 to 25.
1.Tiltable cover, as indicative means of accessibility, without ruling out other means such as sliding surfaces etcetera, via moving of one or more levers(1a) and/or other mechanism-s or otherwise remote controlled suspension mechanism-s (1b) and a cover of freely selected materials, transparent or semi-transparent or ,mnon transparent (1c). The tiltable cover is used for the placement of the device that will be subject to cleaning at the base (5). More covers-access windows for accessibility are possible.
2.Main frame manufactured as independent structure or with the capacity to be modular or with the capacity to expand to all sides and situations. The ability of forming the frame and its adapted parts for the creation of an automated unit is possible. The placement compartments of its cleaning devices and placement of further devices and accessories provide water resistance or limited percentage of water resistance, depending on the version of each Device (Figure1).
3. Access window for the use of flexible materials for the intervention of the user or automated mechanisms on the required cleaning tools or other functions. One of the forms of bendable materials is gloves.
4. Base-s of devices that are to be cleaned or decontaminated or analyzed or combination of the above. May be tiltable or not, perforated or not or accessible in multiple ways with the required parts as well as the required support mechanism-s. Support can be additionally, variable with mechanically changeable or variously automated controlled tilted adaptors in every axis position.
5. Manufacture-s, adaptors, materials for device interconnection, which are used for cleaning, decontamination, analysis or other means with the compartment-s of each device subject to process. The manufacture-s for the adaptors' connection is free and several adaptors are supported controlled mechanically of otherwise, for the interconnection of devices in different compartments or devices.
6. Compartments shaped for the deposition of the parts to absorption and processing. The compartment is free manufacture- and its purpose is the transition of the rest of the equipment of any form of elements (dust etc. which result from the device being processed. The compartment is connected to the compartment(s) where the device being processed is placed and can include individual compartments, conductors or constructions and accessories of any kind.
7. Boards with or without control, programming, and data depiction screen(s), analog or digital to support the respective editions of the Devices (Figure1) such as computer , calculating machines and electric or electronic parts management. The position(s) of placement is/are free to any beneficial place of the construction.
8. Compartment formed for the placement of devices, such as absorption or pressure devices or any devices desired by the manufacturer. The separation and setup between the compartments can be realized by using solid or flexible spacers. Accessibility windows are possible, depending on the design and manufacture.
9. Construction-s or accessories with adaptors for the connection of the devices between compartments.
10. Air filter(s) with cleaning ability, simply or with flow regulation ability, the capacity of one-way or two-way pressure flow and the ability to include gas, liquid, and solid analysis sensors. Free space and number on the whole device for production and edition of each model.
11. Gas, liquid, and solid analysis device-s, with the option of analyzability in real time (free choice of position and number). Multiple additional sensors may exist on the device in preferred places and number.
12. Absorption device-s with the option of ability to eject steam or aquatic particles, and the option of the ability to withhold waste residues in any usable construction and technology (free choice of place and number).
13. Steam ejection or aquatic particle ejection devices as well as several other sensors may exist in the construction and at preferable places and number. Furthermore, any processing or examination can be accomplished outside of the device and insert the channelization or control parts or some of the devices and materials in the device in order for the ultimate goal to be accomplished. Several adjusters and additional sensors may exist in the construction in preferable places and number. The ejection of steam is used as a means of decontamination.
14. Liquids, solids, and gas collecting device-s or a combination of the three collecting devices. Indicatively a type of device is a dispenser that is used for the collection removal, analysis, or sampling analysis of the waste in real or later time, with the option of receiving additional or other way to neutralize - inactivation - and decontaminate before their final management (free choice of place and number). Several adjusters and additional sensors may exist in the construction in optional places and number.
15.Pressure balancers with the purpose to balance pressure in the equipment and areas may exist (free choice of place and number).
16. Air pressure or other gas pressure device-s with or without flow control (free placement and number choice). Various adjusters and additional sensors may exist in the construction in optional place and number.
17. Place of ozone channeling device-s (free choice of place and number).
   Various adjusters and additional sensors may exist in the construction in optional place and number. The ozone device is used as an additional means of decontamination.
18. Place of ionizing device-s (free choice of place and number). The ionizing devices are used as an additional means of decontamination. Various adjusters and additional sensors may exist in the construction in optional place and number.
19. Place-s of device-s with various technologies with the option to manage the visible and invisible spectrum. One of the options is the use of a device with emission in Ultra Violet spectrum as an additional means of decontamination (free choice of place and number).
20. Place-s of device-s which eject(s) and sort of appropriate biological material as an additional means of purification and decontamination (free choice of place and number).
   Various adjusters and additional sensors may exist in the construction in optional place and number.
21. Place-s of device-s which eject-s any sort of chemical material as an additional means of purification and decontamination (free choice of place and number).
   Various adjusters and additional sensors may exist in the construction in optional place and number.
22. Place-s of dehumidification device-s on equipment or space (free choice of place and number). Various adjusters and additional sensors may exist in the construction in optional place and number.
23. Place-s of lighting device-s of the Device (Image 1) (free choice of place and number). Various additional adjusters such as tension, place, and spectrum adjusters as well as mechanisms and sensors may exist in the construction in optional place and number.
24. Place-s of computer, electronic and electric parts. The Device (Image 1) aside from mechanic, electro mechanic electronically, partly or entirely monitored may be partly or entirely automated in its function. Consequently, computers, electronic electric and other parts are respectively included. Some of the equipment is intended to program the equipment, commands and data reception from adjusters, sensors, etc, data recording, mechanism and function management the one-way or two-way function management of other Devices (Image 1) or distant devices via wired or wirelessly through all technologies and indicatively via net or internet.
25. Wheeled or stationary or variable support bases and any form of support bases of the device in general and in any place of the construction.

## Claims

1. System cleaning, decontamination and analysis device for computers, computing machines and their peripherals for use in removal of solids, liquids or gases generated and accumulated in computers, computing machines and their peripherals and result in the creation of areas prone to infection, smells, stressing of the materials, increase of energy consumption, which Device (Figure1) includes means of cleaning decontamination and analysis of waste by accumulation of waste subject to process and management with the option of informative data system which Device (Figure1) indicatively includes a tillable
cover (1) as a means of accessibility which is used for the placement of each device subject to cleaning, decontamination and analysis on the special base (4) and those constitute parts of the construction which consists of a main frame and respective parts (2) manufactured and modified depending on the user's demands with an additional wicket (3) for the user's access or other machines with additional requirements for the interconnection of the devices (5) with additional requirements for the transportation of waste in respective appropriate equipment(6) with depiction machines and computing machines and equipment and management of electric and electronic parts (7) with an in continuation compartment modeled for the placement of absorption devices, air or other gas pressure devices or any other devices (8) with manufactured parts with accessories and adjusters for the interconnection of the devices between spaces (9) with respective air filtering equipment with the ability to be cleaned simply or with flow regulation option, with ability of one way or two ways flow pressure, or with the option to include gas, fluid and solid analysis sensors (10) with gas, fluid and solid analysis equipment even in real time (11) with absorption equipment and the ability to eject steam or aquatic particles and the ability to withhold the waste - residues in any form of construction and technology can be used (12) with ejecting hooting steam or aquatic particles equipment and support of any processing or regulation can be accomplished outside the Device (Figure1) and the Device (Figure1) can incorporate the transportation and monitoring parts or some of the devices and materials for the achievement of the ultimate purpose (13) with fluid, solid, or gas, or combination of the above accumulation equipment for the purpose of accumulation, removal, analysis or sampling analysis of the waste in real or later time with the option of receiving additional assets or other means of neutralization - inactivation decontamination before their final processing (14) with pressure balancing equipment for pressure adjustment between spaces or equipment of the Device (Figure1) and the external environment (15) with air pressure equipment or other pressure equipment appropriate for the gas shooter (16) with ozone supplying equipment as an additional means of disinfection (17) with ionizing equipment as additional means of cleaning and disinfection (18) with visible and invisible spectrum managing equipment while one of the options is the emission in Ultra Violet spectrum as an additional way of decontamination (19) with any kind of biological substance shooting equipment as an additional means of cleaning and decontamination (20) with any kind of chemical substance eject equipment as an additional means of decontamination (21) with humidification equipment in equipment or spaces (22) with lighting equipment (23) with equipment of electronic, computing, or electronic computing machines and other electronic and electric parts because the Device (Figure1) in its function apart from mechanic, electromechanic, partially or entirely electronically controlled can be partially or entirely automated and part of the equipment aims at programming equipment or commands and data reception from adjustors, sensors etcetera, data recording, mechanism and function management the one-way or two-way functions management of other Devices (Figure1) or distant devices wired or wirelessly through all the technologies and indicatively via net or internet (24) with wheeled or stationary or variable support bases and any form or support base in general and in any position they can support it according to each application (25).
